# EUROPEAN PATENT APPLICATION

(11) **EP 4 722 356 A1**
(43) Date of publication of application: **08.04.2026**
(21) Application number: 23938994.3
(22) Date of filing: 02.06.2023
(51) Int. Cl.: C12N 9/12, C12Q 1/68

(54) **DNA POLYMERASE MUTANT FOR SEQUENCING**

(71) Applicant: Wuhan MGI Tech Co., Ltd., Wuhan, Hubei 430075 (CN); MGI Tech Co., Ltd., Shenzhen, Guangdong 518083 (CN)
(72) Inventor: LIU, Fen, Shenzhen, Guangdong 518083 (CN); HUANG, Siqian, Shenzhen, Guangdong 518083 (CN); YANG, Liyuan, Shenzhen, Guangdong 518083 (CN); GAO, Jing, Shenzhen, Guangdong 518083 (CN); BAI, Jiakun, Shenzhen, Guangdong 518083 (CN); XIAO, Yang, Shenzhen, Guangdong 518083 (CN); YANG, Juan, Shenzhen, Guangdong 518083 (CN); SU, Xiang, Shenzhen, Guangdong 518083 (CN); LIU, Yuepeng, Shenzhen, Guangdong 518083 (CN); SONG, Chengwei, Shenzhen, Guangdong 518083 (CN)
(74) Representative: Potter Clarkson
(86) International application number: PCT/CN2023/098118
(87) International publication number: WO 2024/244010

(57) **Abstract**

Provided is a DNA polymerase mutant for sequencing. The DNA polymerase mutant includes: compared with a *Pyrococcus abyssi* DNA polymerase exo-mutant, at least three amino acid mutations in the following four sites or functionally equivalent sites: position 409, position 410, position 411, and position 486. The *Pyrococcus abyssi* DNA polymerase exo-mutant has an amino acid sequence as set forth in SEQ ID NO: 1.

## Description

### FIELD

The present disclosure relates to the field of biotechnology. In particular, the present disclosure relates to a DNA polymerase mutant for sequencing, specifically to a DNA polymerase mutant, a nucleic acid molecule, an expression vector, a recombinant cell, a recombinant strain, a method for producing DNA polymerase mutant, a complex, a method for nucleic acid sequencing, a nucleic acid sequencing kit and use thereof, and use in the preparation of a product for catalyzing DNA amplification or nucleic acid sequencing.

### BACKGROUND

Nucleic acid sequences are crucial for understanding gene function and have been widely applied in gene analysis, and DNA sequencing methods are important tool in gene analysis. Second-generation high-throughput DNA sequencing methods based on sequencing-by-synthesis (SBS) are currently the most widely used sequencing technologies. Compared with first-generation sequencing, second-generation sequencing offers several advantages: high sequencing speed and throughput, enabling large-scale genome sequencing within a short time; low sequencing cost, with the cost per base significantly lower than that of first-generation sequencing; high sequencing accuracy, capable of detecting low-frequency variants and heterozygous sites; and high flexibility, applicable to various target regions and sample types. Second-generation sequencing has broad applications in the biomedical field, including transcriptome sequencing, epigenetic sequencing, genome-wide association studies, drug resistance detection, and other applications such as genetic identification, forensic analysis, genetic counseling, and medical diagnostics.

Sequencing polymerase, as one of the most important components in second-generation sequencing, plays a crucial role in the sequencing process. The principle of second-generation sequencing is sequencing-by-synthesis, i.e., determining the DNA sequence during DNA replication by capturing the special labels (typically fluorescent labels) carried by newly incorporated nucleotides. Sequencing polymerases are responsible for pairing and incorporating labeled nucleotides with the template strand to form the complementary DNA strand. In such sequencing methods, the addition and detection of artificially modified nucleotides or nucleotide analogs (e.g., DNA polymerase with a fluorescently labeled reversible terminator) is essential. In DNA polymerization, a major limiting factor is the low polymerization capability of existing natural polymerases to incorporate artificially modified nucleotides or nucleotide analogs. In view of this, enhancing the polymerase's polymerization capability to incorporate artificially modified nucleotides or nucleotide analogs is a key step in such sequencing methods.

Based on the amino acid sequence differences among *Escherichia coli* (E. coli) DNA polymerases I, II, and III, DNA polymerases are classified into three families, referred to as families A, B, and C, respectively. Although polymerases of families A and B share structurally similar nucleotide-binding sites, the motifs among different polymerase families are significantly distinct and thus represent different recognition mechanisms for nucleotides and their analogs. Among them, thermostable family B polymerases derived from hyperthermophilic archaea exhibit the best incorporation performance for various nucleotide analogs, and their mutants are widely used in various second-generation sequencing methods. Thermostable family B polymerases from hyperthermophilic archaea include KOD (*Thermococcus kodakaraensis*)*,* 9°N (*Thermococcus sp. 9°N*)*,* TGO (*Thermococcus gorgonarius*), TOK (*Desulfurococcus sp. Tok*)*,* Vent DNA polymerase (*Thermococcus litoralis*)*,* JDF-3, and Pfu DNA polymerase (*Pyrococcus furiosus*), among others. The major amino acid sequences at the active sites of these DNA polymerases are conserved, but these conserved enzymatic active sites can be readily modified to incorporate non-natural specific dNTPs while maintaining DNA polymerase activity.

### SUMMARY

The present disclosure is directed, at least in part, to solving at least one of the technical problems existing in the prior art.

In view of the problems existing in current sequencing polymerases with respect to polymerization speed and sequencing quality (e.g., lag (affecting sequencing signal and quality) or strand bias), the inventors performed enzyme engineering modifications on the relevant active sites of thermostable family B polymerases from hyperthermophilic archaea, thereby obtaining novel DNA polymerase mutants. These mutants can significantly improve the incorporation efficiency of polymerases for specific non-natural dNTPs, thereby enhancing both sequencing speed and sequencing quality in sequencing-by-synthesis (SBS) methods.

To this end, in one aspect, the present disclosure provides a DNA polymerase mutant. According to an embodiment of the present disclosure, the mutant includes at least three amino acid mutations at the following four sites or functionally equivalent sites, compared with a *Pyrococcus abyssi* DNA polymerase exo-mutant (in which the 3'-5' exonuclease proofreading activity is removed): position 409, position 410, position 411, and position 486. The *Pyrococcus abyssi* DNA polymerase exo-mutant has an amino acid sequence as set forth in SEQ ID NO: 1. The DNA polymerase mutants described in the embodiments of the present disclosure can effectively improve the incorporation efficiency of polymerases for specific non-natural dNTPs, and thus are of significant importance for improving sequencing speed and sequencing quality in SBS methods.

MIIDADYITEDGKPIIRIFKKEKGEFKVEYDRTFRPYIYALLKDDSAIDEVKK
ITAERHGKIVRITEVEKVQKKFLGRPIEVWKLYLEHPQDVPAIREKIREHPAVVDIFEYD
IPFAKRYLIDKGLTPMEGNEELTFLAVAIATLYHEGEEFGKGPIIMISYADEEGAKVITW
KSIDLPYVEVVSSEREMIKRLVKVIREKDPDVIITYNGDNFDFPYLLKRAEKLGIKLPL
GRDNSEPKMQRMGDSLAVEIKGRIHFDLFPVIRRTINLPTYTLEAVYEAIFGKSKEKVY
AHEIAEAWETGKGLERVAKYSMEDAKVTFELGKEFFPMEAQLARLVGQPVWDVSRS
STGNLVEWFLLRKAYERNELAPNKPDEREYERRLRESYEGGYVKEPEKGLWEGIVSL
DFRSLYPSIIITHNVSPDTLNRENCKEYDVAPQVGHRFCKDFPGFIPSLLGNLLEERQKI
KKRMKESKDPVEKKLLDYRQRAIKILANSYYGYYGYAKARWYCKECAESVTAWGR
QYIDLVRRELESRGFKVLYIDTDGLYATIPGAKHEEIKEKALKFVEYINSKLPGLLELEY
EGFYARGFFVTKKKYALIDEEGKIVTRGLEIVRRDWSEIAKETQAKVLEAILKHGNVD
EAVKIVKEVTEKLSKYEIPPEKLVIYEQITRPLSEYKAIGPHVAVAKRLAAKGVKVKPG
MVIGYIVLRGDGPISKRAIAIEEFDPKKHKYDAEYYIENQVLPAVERILRAFGYRKEDL

KYQKTKQVGLGAWLKF (SEQ ID NO: 1).

It should be noted that the amino acid positions in the amino acid sequences of the DNA polymerase mutants described herein are determined with reference to the positions of amino acids in the amino acid sequences of the wild-type *Pyrococcus abyssi* DNA polymerase or wild-type *Pyrococcus abyssi* DNA polymerase exo-mutant.

According to an embodiment of the present disclosure, the above-mentioned DNA polymerase mutant may further include at least one of the following additional technical features:

According to an embodiment of the present disclosure, the DNA polymerase mutant has at least 90% identity to the *Pyrococcus abyssi* DNA polymerase exo-mutant.

According to an embodiment of the present disclosure, the mutant has the following mutations:
(1) amino acid L at position 409 is mutated to A, V, Y, H, F, or Q;
(2) amino acid Y at position 410 is mutated to A or G;
(3) amino acid P at position 411 is not mutated or is mutated to V, G, C, or S; and
(4) amino acid A at position 486 is mutated to I, K, L, M, W, or F.

According to an embodiment of the present disclosure, the mutant has any one of mutation combinations (1) to (2):
(1) amino acid Y at position 410 is mutated to A, amino acid L at position 409 is mutated to A, V, Y, H, F, or Q, amino acid P at position 411 is mutated to V, G, C, or S or is not mutated, and amino acid A at position 486 is mutated to I, K, L, M, W, or F; or
(2) amino acid Y at position 410 is mutated to G, amino acid L at position 409 is mutated to A, V, Y, H, F, or Q, amino acid P at position 411 is mutated to V, G, C, or S or is not mutated, and amino acid A at position 486 is mutated to I, K, L, M, W, or F.

According to an embodiment of the present disclosure, the mutant has any one of mutation combinations (1) to (7):
(1) amino acid Y at position 410 is mutated to A, amino acid L at position 409 is mutated to A, amino acid P at position 411 is mutated to G, C, or S or is not mutated, and amino acid A at position 486 is mutated to I, K, L, M, W, or F;
(2) amino acid Y at position 410 is mutated to A, amino acid L at position 409 is mutated to V, amino acid P at position 411 is mutated to V, G, or C or is not mutated, and amino acid A at position 486 is mutated to I, L, W, or F;
(3) amino acid Y at position 410 is mutated to A, amino acid L at position 409 is mutated to Y, amino acid P at position 411 is mutated to V or G or is not mutated, and amino acid A at position 486 is mutated to L;
(4) amino acid Y at position 410 is mutated to A, amino acid L at position 409 is mutated to H, amino acid P at position 411 is mutated to V or G or is not mutated, and amino acid A at position 486 is mutated to L;
(5) amino acid Y at position 410 is mutated to A, amino acid L at position 409 is mutated to F, amino acid P at position 411 is mutated to V or G or is not mutated, and amino acid A at position 486 is mutated to L;
(6) amino acid Y at position 410 is mutated to A, amino acid L at position 409 is mutated to Q, amino acid P at position 411 is mutated to G or is not mutated, and amino acid A at position 486 is mutated to L; or
(7) amino acid Y at position 410 is mutated to G, amino acid L at position 409 is mutated to V, amino acid P at position 411 is not mutated, and amino acid A at position 486 is mutated to I, K, M, or F.

According to some embodiments of the present disclosure, sequencing recombinant enzymes (obtained from the DNA polymerase mutants of the present disclosure) generated at different mutation sites exhibit different relative activities, providing more options for practical production.

According to an embodiment of the present disclosure, the mutant has any one of mutation combinations (1) to (36):
(1) L at position 409 is mutated to A, Y at position 410 is mutated to A, P at position 411 is not mutated, and A at position 486 is mutated to I;
(2) L at position 409 is mutated to V, Y at position 410 is mutated to A, P at position 411 is not mutated, and A at position 486 is mutated to L;
(3) L at position 409 is mutated to A, Y at position 410 is mutated to A, P at position 411 is not mutated, and A at position 486 is mutated to W;
(4) L at position 409 is mutated to V, Y at position 410 is mutated to G, P at position 411 is not mutated, and A at position 486 is mutated to K;
(5) L at position 409 is mutated to V, Y at position 410 is mutated to G, P at position 411 is not mutated, and A at position 486 is mutated to I;
(6) L at position 409 is mutated to A, Y at position 410 is mutated to A, P at position 411 is not mutated, and A at position 486 is mutated to M;
(7) L at position 409 is mutated to V, Y at position 410 is mutated to G, P at position 411 is not mutated, and A at position 486 is mutated to F;
(8) L at position 409 is mutated to V, Y at position 410 is mutated to A, P at position 411 is mutated to V, and A at position 486 is mutated to L;
(9) L at position 409 is mutated to A, Y at position 410 is mutated to A, P at position 411 is not mutated, and A at position 486 is mutated to F;
(10) L at position 409 is mutated to A, Y at position 410 is mutated to A, P at position 411 is mutated to G, and A at position 486 is mutated to I;
(11) L at position 409 is mutated to A, Y at position 410 is mutated to A, P at position 411 is mutated to C, and A at position 486 is mutated to I;
(12) L at position 409 is mutated to Y, Y at position 410 is mutated to A, P at position 411 is not mutated, and A at position 486 is mutated to L;
(13) L at position 409 is mutated to A, Y at position 410 is mutated to A, P at position 411 is mutated to S, and A at position 486 is mutated to K;
(14) L at position 409 is mutated to Y, Y at position 410 is mutated to A, P at position 411 is mutated to G, and A at position 486 is mutated to L;
(15) L at position 409 is mutated to Y, Y at position 410 is mutated to A, P at position 411 is mutated to V, and A at position 486 is mutated to L;
(16) L at position 409 is mutated to H, Y at position 410 is mutated to A, P at position 411 is not mutated, and A at position 486 is mutated to L;
(17) L at position 409 is mutated to F, Y at position 410 is mutated to A, P at position 411 is not mutated, and A at position 486 is mutated to L;
(18) L at position 409 is mutated to A, Y at position 410 is mutated to A, P at position 411 is not mutated, and A at position 486 is mutated to L;
(19) L at position 409 is mutated to A, Y at position 410 is mutated to A, P at position 411 is mutated to C, and A at position 486 is mutated to M;
(20) L at position 409 is mutated to V, Y at position 410 is mutated to G, P at position 411 is not mutated, and A at position 486 is mutated to M;
(21) L at position 409 is mutated to A, Y at position 410 is mutated to A, P at position 411 is mutated to G, and A at position 486 is mutated to W;
(22) L at position 409 is mutated to A, Y at position 410 is mutated to A, P at position 411 is mutated to G, and A at position 486 is mutated to M;
(23) L at position 409 is mutated to V, Y at position 410 is mutated to A, P at position 411 is not mutated, and A at position 486 is mutated to W;
(24) L at position 409 is mutated to V, Y at position 410 is mutated to A, P at position 411 is mutated to C, and A at position 486 is mutated to L;
(25) L at position 409 is mutated to Q, Y at position 410 is mutated to A, P at position 411 is mutated to G, and A at position 486 is mutated to L;
(26) L at position 409 is mutated to H, Y at position 410 is mutated to A, P at position 411 is mutated to G, and A at position 486 is mutated to L;
(27) L at position 409 is mutated to A, Y at position 410 is mutated to A, P at position 411 is mutated to S, and A at position 486 is mutated to F;
(28) L at position 409 is mutated to V, Y at position 410 is mutated to A, P at position 411 is not mutated, and A at position 486 is mutated to I;
(29) L at position 409 is mutated to H, Y at position 410 is mutated to A, P at position 411 is mutated to V, and A at position 486 is mutated to L;
(30) L at position 409 is mutated to A, Y at position 410 is mutated to A, P at position 411 is mutated to S, and A at position 486 is mutated to I;
(31) L at position 409 is mutated to A, Y at position 410 is mutated to A, P at position 411 is mutated to S, and A at position 486 is mutated to M;
(32) L at position 409 is mutated to F, Y at position 410 is mutated to A, P at position 411 is mutated to G, and A at position 486 is mutated to L;
(33) L at position 409 is mutated to V, Y at position 410 is mutated to A, P at position 411 is mutated to G, and A at position 486 is mutated to L;
(34) L at position 409 is mutated to V, Y at position 410 is mutated to A, P at position 411 is not mutated, and A at position 486 is mutated to F;
(35) L at position 409 is mutated to A, Y at position 410 is mutated to A, P at position 411 is mutated to G, and A at position 486 is mutated to F; or
(36) L at position 409 is mutated to F, Y at position 410 is mutated to A, P at position 411 is mutated to V, and A at position 486 is mutated to L.

According to an embodiment of the present disclosure, the mutant has the following mutations:
(1) L at position 409 is mutated to V, Y at position 410 is mutated to G, P at position 411 is not mutated, and A at position 486 is mutated to I;
(2) L at position 409 is mutated to A, Y at position 410 is mutated to A, P at position 411 is not mutated, and A at position 486 is mutated to I;
(3) L at position 409 is mutated to A, Y at position 410 is mutated to A, P at position 411 is not mutated, and A at position 486 is mutated to M;
(4) L at position 409 is mutated to A, Y at position 410 is mutated to A, P at position 411 is not mutated, and A at position 486 is mutated to L;
(5) L at position 409 is mutated to V, Y at position 410 is mutated to A, P at position 411 is not mutated, and A at position 486 is mutated to I; or
(6) L at position 409 is mutated to A, Y at position 410 is mutated to A, P at position 411 is mutated to C, and A at position 486 is mutated to F.

According to some embodiments of the present disclosure, sequencing recombinant enzymes (obtained from the DNA polymerase mutants of the present disclosure) generated at different mutation sites exhibit different relative activities, providing more options for practical production.

It should be noted that the amino acid sequences of the above mutants are obtained by introducing mutations into the sequence of SEQ ID NO: 1.

According to some embodiments of the present disclosure, when the amino acid sequence of the DNA polymerase mutant includes the above-mentioned mutations, the resulting recombinant polymerase exhibits higher relative activity compared to recombinant polymerases obtained from existing mutations, enabling effective incorporation of non-natural dNTPs containing a fluorescent labeled 3'O-reversible terminator into a DNA strand, thereby improving sequencing speed and sequencing quality.

It should be noted that the wild-type *Pyrococcus abyssi* DNA polymerase is unable to incorporate specific non-natural dNTPs (e.g., non-natural dNTPs containing a 3'O-reversible terminator) into a DNA strand. Accordingly, the inventors have designed a *Pyrococcus abyssi* DNA polymerase mutant that is capable of incorporating such specific non-natural dNTPs into a DNA strand.

In a second aspect of the present disclosure, the present disclosure provides a nucleic acid molecule. According to an embodiment of the present disclosure, the nucleic acid molecule encodes the DNA polymerase mutant according to the first aspect of the present disclosure. The DNA polymerase mutant encoded by the nucleic acid molecule can be obtained in large quantities either *in vivo* or *in vitro.*

In a third aspect of the present disclosure, the present disclosure provides an expression vector. According to an embodiment of the present disclosure, the expression vector includes the nucleic acid molecule according to the second aspect of the present disclosure.

It should be noted that the expression vector may further include a promoter operably linked to the nucleic acid molecule.

According to an embodiment of the present disclosure, the expression vector is a non-pathogenic viral vector. The non-pathogenic viral vector includes an adenoviral vector or a retroviral vector.

In a fourth aspect of the present disclosure, the present disclosure provides a recombinant cell. According to an embodiment of the present disclosure, the recombinant cell carries the nucleic acid molecule according to the second aspect of the present disclosure or the expression vector according to the third aspect of the present disclosure. The recombinant cell is used to express or secrete the DNA polymerase mutant according to the first aspect of the present disclosure.

According to an embodiment of the present disclosure, the recombinant cell is selected from *Escherichia coli,* yeast, or mammalian cells.

In a fifth aspect of the present disclosure, the present disclosure provides a recombinant strain. According to an embodiment of the present disclosure, the recombinant strain expresses the DNA polymerase mutant according to the first aspect of the present disclosure. By culturing the recombinant strain, the DNA polymerase mutant can be rapidly obtained in large quantities.

In a sixth aspect of the present disclosure, the present disclosure provides a method for obtaining a DNA polymerase mutant. According to an embodiment of the present disclosure, the method includes culturing the recombinant cell according to the fourth aspect of the present disclosure or the recombinant strain according to the fifth aspect of the present disclosure under conditions suitable for protein expression, to obtain the DNA polymerase mutant.

In a seventh aspect of the present disclosure, the present disclosure provides a complex. According to an embodiment of the present disclosure, the complex includes the DNA polymerase mutant according to the first aspect of the present disclosure and a small molecule compound or a macromolecule. The DNA polymerase mutant is conjugated, via a chemical bond, with the small molecule compound or the macromolecule.

According to an embodiment of the present disclosure, the small molecule compound or the macromolecule includes a fluorescent label, fluorescein, an antibody, or the like.

In an eighth aspect of the present disclosure, the present disclosure provides a method for nucleic acid synthesis. According to an embodiment of the present disclosure, the method includes: subjecting a mixture of a nucleic acid template, an amplification primer, dNTPs, and the DNA polymerase mutant of the first aspect of the present disclosure under conditions suitable for nucleic acid amplification, to obtain the nucleic acid. Rapid and efficient amplification of nucleic acid templates can be achieved using the aforementioned nucleic acid synthesis method.

It should be noted that the DNA polymerase mutants described in this application exhibit polymerization activity for all dNTPs, including both fluorescently labeled and non-fluorescently labeled dNTPs.

In a ninth aspect of the present disclosure, the present disclosure provides a method for nucleic acid sequencing. According to an embodiment of the present disclosure, the method includes: subjecting a mixture of a nucleic acid to be sequenced, the DNA polymerase mutant according to the first aspect of the present disclosure, and non-natural dNTPs with a fluorescently labeled 3'O-reversible terminator to amplification under conditions suitable for nucleic acid amplification and fluorescence signal detection; and determining the nucleotide sequence of the nucleic acid to be sequenced based on the detected fluorescence signal.

By way of example, the nucleic acid sequencing method further includes mixing the nucleic acid to be sequenced with the DNA polymerase mutant and non-natural dNTPs with a fluorescently labeled 3'O-reversible terminator. The DNA polymerase mutant is responsible for incorporating non-natural dNTPs with a fluorescently labeled 3'O-reversible terminator into the nucleic acid in a base-complementary manner. Finally, the nucleic acid sequence of the nucleic acid to be sequenced is obtained by detecting the plurality of fluorescent signals and analyzing the nucleic acid sequence derived from the obtained fluorescent signals. The above polymerization reaction and fluorescence detection can be repeated in multiple cycles according to the length of the sequencing template.

In a tenth aspect of the present disclosure, the present disclosure provides a nucleic acid sequencing kit. According to an embodiment of the present disclosure, the nucleic acid sequencing kit includes the DNA polymerase mutant according to the first aspect or the complex according to the seventh aspect. The kit of the present disclosure is useful for efficient, accurate, and rapid nucleic acid sequencing.

In an eleventh aspect of the present disclosure, the present disclosure provides use of the nucleic acid sequencing kit according to the ninth aspect in sequencing. According to an embodiment of the present disclosure, the kit can be used for sequencing, including but not limited to sequencing-by-synthesis (SBS).

In a twelfth aspect of the present disclosure, the present disclosure provides use of the DNA polymerase mutant according to the first aspect, the nucleic acid molecule according to the second aspect, the expression vector according to the third aspect, the recombinant cell according to the fourth aspect, the recombinant strain according to the fifth aspect, or the complex according to the seventh aspect in the preparation of a product for catalyzing DNA amplification or nucleic acid sequencing. According to an embodiment of the present disclosure, the DNA polymerase mutant, nucleic acid molecule, expression vector, recombinant cell, recombinant strain, or complex may be used alone or in combination in the preparation of a product for catalyzing DNA amplification or nucleic acid sequencing.

It is to be understood that within the scope of the present disclosure, the technical features described above and the technical features specifically described hereinafter (such as embodiments) may be combined with one another to form new or preferred technical solutions. Due to space limitations, they are not exhaustively described herein.

### BRIEF DESCRIPTION OF DRAWINGS

The above and/or additional aspects and advantages of the present disclosure will become apparent and more readily understood from the following description of embodiments with reference to the accompanying drawings, in which:

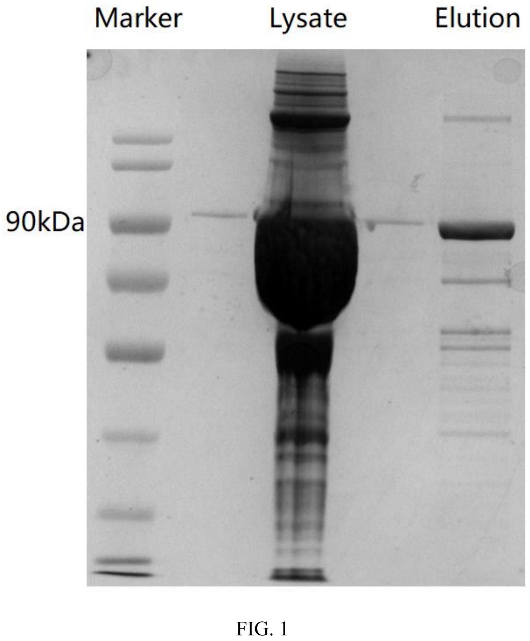

FIG. 1 shows an electropherogram of the *Pyrococcus abyssi* exo-type DNA polymerase fusion protein described in Example 1 of the present disclosure.

### DETAILED DESCRIPTION

Embodiments of the present disclosure are described in detail below, examples of which are illustrated in the accompanying drawings. The embodiments described below with reference to the drawings are exemplary and are intended to explain the present disclosure, and are not to be construed as limiting the present disclosure.

### Definitions and explanations

Furthermore, the terms "first", "second" and the like are used for descriptive purposes only and are not to be construed as indicating or implying relative importance or as implicitly indicating the number of the referenced technical features. Thus, features defined as "first", "second" or the like may explicitly or implicitly include at least one such feature. In the description of the present disclosure, "a plurality" or "more" means at least two, e.g., two, three, and so on, unless otherwise specifically defined.

In the present application, the amino acid sequence as set forth in SEQ ID NO: 1 is obtained by mutating amino acid D at position 141 to A and amino acid E at position 143 to A of the wild-type *Pyrococcus abyssi* DNA polymerase. The wild-type *Pyrococcus abyssi* DNA polymerase exhibits no exonuclease activity.

In the present application, unless otherwise specified, the terms "identity" and "homology" have the same meaning.

It is an object of the present disclosure to provide a family B recombinant polymerase capable of improving sequencing quality. This enzyme is a DNA polymerase based on the archaeal *Pyrococcus abyssi* (Erauso, Gaël, et al. "Pyrococcus abyssi sp. nov., a new hyperthermophilic archaeon isolated from a deep-sea hydrothermal vent." Archives of microbiology 160 (1993): 338-349), characterized by high activity and high thermostability, but the wild-type polymerase does not efficiently incorporate non-natural dNTPs with a fluorescent label and a 3'O-reversible terminator. Enzymatic engineering modification and screening on relevant active sites is required to improve the enzyme's incorporation efficiency of specific non-natural dNTPs for use in SBS methods.

### DNA polymerase mutant

The present disclosure provides a DNA polymerase mutant. The DNA polymerase mutant provided in the embodiments of the present disclosure has an amino acid sequence at least 90% homologous to the wild-type *Pyrococcus abyssi* DNA polymerase exo-mutant and includes mutations at at least three of the following positions: position 409, position 410, position 411, and position 486.

The DNA polymerase mutant described in the present application offers the advantages of high activity and high thermostability, and exhibits improved performance in polymerizing non-natural dNTPs with a 3'O-reversible terminator, thereby enhancing sequencing speed and quality.

### Nucleic acid molecule

According to some embodiments of the present disclosure, a nucleic acid molecule encoding the aforementioned DNA polymerase mutant is provided.

It should be noted that, with respect to the nucleic acids referred to in the specification and claims of the present disclosure, a person skilled in the art would understand that either one or both strands of the complementary double-stranded nucleic acid are included. For convenience, in this specification and claims, although only one strand is typically presented, the complementary strand is also disclosed. In addition, the nucleic acid sequences in this application include DNA or RNA forms; the disclosure of one form implies the disclosure of the other.

### Expression vector

The present disclosure provides an expression vector including the nucleic acid molecule described above. The type of the expression vector is not particularly limited, provided that it is capable of replicating and expressing the corresponding mutant in a host cell.

### Recombinant cell

The present disclosure provides a recombinant cell carrying the nucleic acid molecule or expression vector, or expressing the DNA polymerase mutant described above. The recombinant cell is obtained by transfection or transformation of the expression vector. According to some embodiments of the present disclosure, the recombinant cell highly expresses the DNA polymerase mutant described above under suitable conditions.

### Recombinant strain

The present disclosure provides a recombinant strain expressing the DNA polymerase mutant described above. The recombinant strain can rapidly propagate within a short period of time and enable efficient production of the DNA polymerase mutant.

### Method for obtaining DNA polymerase mutant

The method for obtaining a DNA polymerase mutant according to the present disclosure includes culturing the recombinant cell described above under conditions suitable for protein expression to obtain the DNA polymerase mutant.

### Complex

The present disclosure provides a complex including the aforementioned DNA polymerase mutant and a small molecule compound or a macromolecule. The DNA polymerase mutant is conjugated, via a chemical bond, with the small molecule compound or the macromolecule. In the field of gene sequencing, the nucleic acid sequence to be sequenced is determined by detecting the small molecule compound (fluorescent label) or macromolecule (antibody or fluorescein) in the complex.

The present disclosure is now described with reference to specific embodiments. It should be understood that these embodiments are for illustrative purposes only and are not intended to limit the scope of the present disclosure in any way.

### Example 1

According to an embodiment of the present disclosure, DNA polymerase and its mutants based on *Pyrococcus abyssi* exo-type (exo-mutant) were prepared and subjected to preliminary enzymatic activity screening. The specific steps are as follows:

### 1.1 Construction and transformation of recombinant expression vector

Sangon Biotech (Shanghai) Co., Ltd. synthesized the *Pyrococcus abyssi* exo-type DNA polymerase gene (SEQ ID NO: 1) fused at the C-terminus with a 6×His tag, cloned into the pD441-WT vector, as well as site-directed mutagenesis primers targeting positions 409, 410, 411, and 485.

The primers were designed as follows: 5'-reverse complementary region (15-21 bp)-non-complementary region (at least 15 bp). The target plasmid was amplified using Phanta Max Super-Fidelity DNA Polymerase.

Positions 409, 410, 411, and 485 were mutated to the following amino acids:
L409 was mutated to A, F, H, L, Q, S, V, or Y;
Y410 was mutated to A or G;
P411 was mutated to A, C, E, F, H, I, K, L, M, N, P, Q, R, S, T, V, W, or Y;
A485 was mutated to F, I, L, K, M, or W.

Among currently known family B DNA polymerases (such as 9°N, Pfu, Vent, etc.), a mutant combination exhibiting high activity for polymerizing non-natural dNTPs with a 3'O-reversible terminator includes L409A-Y410A-P411I-A486L in 9°N polymerase mutant (see EP1664287B1 and US8460910B2). This mutant combination was used as the control mutant site combination (SEQ ID NO: 2) in the *Pyrococcus abyssi* polymerase screening experiments.

MIIDADYITEDGKPIIRIFKKEKGEFKVEYDRTFRPYIYALLKDDSAIDEVKK
ITAERHGKIVRITEVEKVQKKFLGRPIEVWKLYLEHPQDVPAIREKIREHPAVVDIFEYD
IPFAKRYLIDKGLTPMEGNEELTFLAVAIATLYHEGEEFGKGPIIMISYADEEGAKVITW
KSIDLPYVEVVSSEREMIKRLVKVIREKDPDVIITYNGDNFDFPYLLKRAEKLGIKLPL
GRDNSEPKMQRMGDSLAVEIKGRIHFDLFPVIRRTINLPTYTLEAVYEAIFGKSKEKVY
AHEIAEAWETGKGLERVAKYSMEDAKVTFELGKEFFPMEAQLARLVGQPVWDVSRS
STGNLVEWFLLRKAYERNELAPNKPDEREYERRLRESYEGGYVKEPEKGLWEGIVSL
DFRSAAISIIITHNVSPDTLNRENCKEYDVAPQVGHRFCKDFPGFIPSLLGNLLEERQKI
KKRMKESKDPVEKKLLDYRQRLIKILANSYYGYYGYAKARWYCKECAESVTAWGR
QYIDLVRRELESRGFKVLYIDTDGLYATIPGAKHEEIKEKALKFVEYINSKLPGLLELEY
EGFYARGFFVTKKKYALIDEEGKIVTRGLEIVRRDWSEIAKETQAKVLEAILKHGNVD

EAVKIVKEVTEKLSKYEIPPEKLVIYEQITRPLSEYKAIGPHVAVAKRLAAKGVKVKPG
MVIGYIVLRGDGPISKRAIAIEEFDPKKHKYDAEYYIENQVLPAVERILRAFGYRKEDL
KYQKTKQVGLGAWLKF (SEQ ID NO: 2).

Partial mutation combinations at the four sites in the enzyme mutants is shown in Table 1, where the control mutant Mut1 combination corresponds to L409A-Y410A-P4111-A486L.

**Table 1:**

| **Mutant** | **L409** | **Y410** | **P411** | **A486** | **Relative Activity (Relative Vmax/Km)** |
|---|---|---|---|---|---|
| WT | L | Y | P | A | 0.00 |
| Mut1 | A | A | I | L | 1.00 |
| Mut655 | A | A | P | I | 2.26 |
| Mut600 | V | A | P | L | 2.15 |
| Mut654 | A | A | P | W | 2.05 |
| Mut705 | V | G | P | K | 2.00 |
| Mut704 | V | G | P | I | 1.91 |
| Mut657 | A | A | P | M | 1.88 |
| Mut707 | V | G | P | F | 1.85 |
| Mut601 | V | A | V | L | 1.82 |
| Mut658 | A | A | P | F | 1.78 |
| Mut665 | A | A | G | I | 1.52 |
| Mut670 | A | A | C | I | 1.51 |
| Mut612 | Y | A | P | L | 1.48 |
| Mut676 | A | A | S | K | 1.46 |
| Mut614 | Y | A | G | L | 1.43 |
| Mut613 | Y | A | V | L | 1.42 |
| Mut616 | H | A | P | L | 1.40 |
| Mut620 | F | A | P | L | 1.40 |
| Mut83 | A | A | P | L | 1.32 |
| Mut672 | A | A | C | M | 1.32 |
| Mut706 | V | G | P | M | 1.31 |
| Mut664 | A | A | G | W | 1.29 |
| Mut667 | A | A | G | M | 1.28 |
| Mut708 | V | A | P | W | 1.28 |
| Mut603 | V | A | C | L | 1.26 |
| Mut610 | Q | A | G | L | 1.25 |
| Mut618 | H | A | G | L | 1.25 |
| Mut678 | A | A | S | F | 1.22 |
| Mut709 | V | A | P | I | 1.22 |
| Mut617 | H | A | V | L | 1.17 |
| Mut675 | A | A | S | I | 1.17 |
| Mut677 | A | A | S | M | 1.17 |
| Mut622 | F | A | G | L | 1.14 |
| Mut602 | V | A | G | L | 1.11 |
| Mut712 | V | A | P | F | 1.09 |
| Mut668 | A | A | G | F | 1.05 |
| Mut621 | F | A | V | L | 1.03 |
| Mut671 | A | A | C | K | 0.94 |
| Mut619 | H | A | C | L | 0.86 |
| Mut673 | A | A | C | F | 0.86 |
| Mut623 | F | A | C | L | 0.82 |
| Mut611 | Q | A | C | L | 0.71 |
| Mut75 | A | A | Q | L | 0.63 |
| Mut669 | A | A | C | W | 0.52 |
| Mut84 | A | A | N | L | 0.28 |
| Mut87 | A | A | H | L | 0.05 |
| Mut50 | A | A | W | L | 0.00 |
| Mut82 | A | A | Y | L | 0.00 |
| Mut80 | A | A | F | L | 0.00 |

Since the amplification product contained the original template plasmid, in order to avoide false-positive transformants after transformation, Dpn I digestion was performed prior to recombinant cyclization to remove methylated template plasmids.

The recombinant polymerase mutant expression vector was introduced into *E. coli* BL21 competent cells and plated on selective plates (containing kanamycin 50 µg/ml) to screen for positive colonies. After overnight culture, single colonies from the experimental groups were inoculated into 5 ml LB culture medium (containing kanamycin 50 µg/ml) and cultured at 37°C, 200 rpm overnight. On the following day, the culture was diluted 1:100 and inoculated into 96-well plates containing 150 µl fresh LB medium (containing kanamycin 50 µg/ml), followed by incubation at 37°C with shaking at 220 rpm until OD600 = 0.6.

### 1.2 Induction of recombinant protein expression and cell lysis

In the 96-well plates from step 1.1, IPTG (isopropyl β-D-1-thiogalactopyranoside) was added to a final concentration of 0.5 mM, followed by incubation at 25°C and 220 rpm/min for 12-16 h to induce protein expression. Then, 1 µL of 100 mM PMSF (protease inhibitor) and 5 µL of 10 mg/ml lysozyme were added. The mixture was gently pipetted up and down to ensure mixing, followed by incubation at 37°C for 10 min, thereby obtaining the crude cell lysate containing the enzyme.

### 1.3 FRET assay for recombinant protein and incorporation efficiency of non-natural dNTPs

AF532 fluorescent dye-labeled 3'-O-blocked modified dATP (BGI patent US10988501B2) and Cy5 fluorescent dye-labeled DNA strand (Sangon Biotech (Shanghai) Co., Ltd.) were added into the crude cell lysate obtained in step 1.2. The sequence information was as follows:
F: Cy5-CGTGTATGCGTAATAGGATCCCGACTCACTATGGACG (SEQ ID NO: 3);
R: Cy5-CGTGTATCGTCCATAGTGAGTCGGGATCCTATTACGC (SEQ ID NO: 4).

The incorporation of modified nucleotides during high-throughput sequencing was simulated. The relative reaction rates of polymerase mutants were determined using a microplate reader, and the crude enzymatic activity was calculated. The specific experimental methods were as follows:
Reaction buffer: 20 mM Tris-HCl, 10 mM (NH₄)₂SO₄,10 mM KCl, 2 mM MgSO₄, pH 8.5 at 25°C;
Reaction system: 1 µg of different polymerase proteins obtained in step 1.2 were each mixed with a reaction solution containing 2 µM modified dATP and 1 µM DNA-Cy5;
Reaction solution composition: 20 mM Tris-HCl, 10 mM (NH₄)₂SO₄, 10 mM KCl, 2 mM MgSO₄,2 µM 3'-O-blocked modified dATP, 1 µM DNA-Cy5, balance water, pH 8.5;
Reaction temperature: 60°C;
Reaction time: 1 h.

After completion of the reaction, data tables and enzyme activity curves were exported, and the relative activity (Relative Vmax/Km) of the crude enzyme was calculated by comparing with the control enzyme mutant P1. Partial results are shown in Table 1. The wild-type *Pyrococcus abyssi* exo-DNA polymerase P0 (WT-Exo-) failed to incorporate the 3'-O-blocked modified dATP labeled with AF532 fluorescent dye, whereas some mutant proteins were capable of incorporation and generated fluorescence signals. From the experimental results, it can be seen that different mutants exhibited varying polymerization activities toward the modified 3'-blocked dNTPs, indicating effective mutations and site combinations at different sites. Mutant site combinations with Relative Vmax/Km values higher than that of the control were identified as mutants capable of efficiently incorporating 3'-blocked dNTPs. These results suggest that the identified mutants hold significant potential for application in high-throughput sequencing.

### Example 2

According to an embodiment of the present disclosure, expression and purification of *Pyrococcus abyssi* exo-type DNA polymerase mutant fusion proteins were carried out. The specific steps are as follows:
1) A single colony of the BL211pD441 mutant was picked and inoculated into 50 ml of LB liquid culture medium (containing kanamycin 50 µg/ml) and cultured overnight at 37°C, 220 rpm/min. On the following day, the culture was diluted 1:100 and inoculated into 1000 ml of LB medium (containing kanamycin 50 µg/ml), followed by incubation at 37°C with shaking at 220 rpm until OD600 = 0.5-0.8. IPTG was added to a final concentration of 0.5 mM, followed by induction at 25°C overnight. The induced BL21/pD441 cells were then harvested. Meanwhile, a blank control without IPTG addition was set up, and the non-induced BL21/pD441 cells were harvested.
2) The induced BL211pD441 cells from step 1) were centrifuged at 8000 rpm for 10 min. The supernatant was discarded, and the cell pellet was collected and resuspended in Buffer 1 (50 mM KPO₄, 500 mM NaCl, 10 mM imidazole, 5% glycerol, pH 7.0) supplemented with PMSF (final concentration 0.5 mM), Triton X-100 (final concentration 0.5%), and lysozyme (final concentration 0.25%). The suspension was incubated at room temperature for 30 min, centrifuged at 12000 rpm for 30 min at 4°C, and subjected to an ice bath and ultrasonication, followed by centrifugation at 12000 rpm for 30 min. The resulting supernatant was incubated in a water bath at 75°C for 20 min with periodic mixing to ensure uniform heating, and centrifuged at 12000 rpm/min for 30 min at 4°C. The supernatant was filtered through a 0.22 µm membrane to obtain the crude fusion protein extract.
3) The crude fusion protein extract obtained in step 2) was loaded onto a Ni-affinity chromatography column (HisTrap FF, 5 ml, 17-5255-01, GE Healthcare) at an appropriate flow rate. After sample loading, the column was equilibrated with 5 CV (column volumes) of Buffer 1 and then eluted with 5 CV of 3% Buffer 2 (50 mM KPO₄, 1 M NaCl, 5% glycerol, pH 7.0) followed by 5 CV of 50% Buffer 2. The Ni-affinity chromatography eluate corresponding to the peak value greater than or equal to 100 mAU was collected.
4) The eluate corresponding to the peak value of greater than or equal to 100 mAU was loaded onto an ion exchange chromatography column (HiTrap Q HP, 5 ml, 17-1154-01, GE Healthcare) at a certain flow rate. The column was then equilibrate with 5 CV of Buffer 2, followed by linear elution with a gradient from 0% to 60% Buffer 2. The ion exchange chromatography eluate corresponding to the peak value greater than or equal to 100 mAU was collected.
5) The ion exchange chromatography eluate corresponding to the peak value greater than or equal to 100 mAU was subjected to gel filtration chromatography (HiPrep Sephacryl S-100HR, 26 mm, 17-1194-01, GE Healthcare). The column was first washed with 3 CV of 20% ethanol followed by 3 CV of water, AND then equilibrated with 3 CV of 100% Buffer 3 (20 mM Tris, 200 mM KCl, 0.2 mM EDTA, 10% glycerol, pH 7.4). The samples were loaded and eluted with 1.5 CV of Buffer 3. The eluate collected corresponded to the purified wild-type DNA polymerase fusion protein.
6) The *Pyrococcus abyssi* exo-type DNA polymerase fusion protein was subjected to SDS-PAGE (5% stacking gel, 12% separating gel). The results are shown in FIG. 1. Lane 1: protein marker (PageRuler Prestained Protein Ladder, 26616, Thermo Scientific); Lane 2: 1 µl of supernatant from crude fusion protein extract; Lane 3: 1 µl of 1 mg/ml *Pyrococcus abyssi* exo-type DNA polymerase mutant fusion protein purified by Ni-affinity chromatography. As can be seen, the proteins in lanes 2 and 3 were approximately 90 KDa, consistent with the molecular weight reported in the literature (Dietrich, J., Schmitt, P., Zieger, M., Preve, B., Rolland, J. L., Chaabihi, H., & Gueguen, Y. (2002). PCR performance of the highly thermostable proof-reading B-type DNA polymerase from Pyrococcus abyssi. FEMS microbiology letters, 217(1), 89-94.). Protein purity was analyzed using Quantity One software on the electrophoresis gel, showing that the purity of the purified DNA polymerase mutant fusion protein reached 85% or higher. The target protein of approximately 90 KDa was not observed in the non-induced BL211pD441 cells.

### Example 3

According to an embodiment of the present disclosure, the actual sequencing performance of the purified DNA polymerase mutant enzyme based on the *Pyrococcus abyssi* exo-type was evaluated on a sequencer. Specifically, the purified enzyme of the advantageous mutant was subjected to sequencing on the MGISEQ-2000 sequencer of BGI, and the sequencing results were analyzed. The specific experimental steps are as follows:
Sequencing library: *E. coli.fa* library (Part No. 1000005038, MGI Tech Co., Ltd);
Sequencing conditions: polymerization time of 90 seconds and cleavage time of 60 seconds.

Using the same sequencing library *E. coli.fa* library and sequencing conditions, the control P1 mutant and five mutants that exhibited varying performance in the enzymatic activity assay were selected for on-instrument testing. These mutants were subjected to 100 sequencing cycles (SE100) on the MGISEQ-2000 sequencer. The test results are shown in Table 2, indicating that the purified enzymes of the advantageous mutants exhibited superior performance in actual sequencing.

**Table 2:**

| Items | Mut1 | Mut83 | Mut655 | Mut657 | Mut673 | Mut704 | Mut709 |
|---|---|---|---|---|---|---|---|
| Q30 (%) | 89.94 | 92.05 | 91.60 | 94.10 | 88.39 | 95.30 | 93.50 |
| Avg Error Rate (%) | 0.47 | 0.26 | 0.33 | 0.24 | 0.65 | 0.19 | 0.29 |
| Lag (%) | 0.24 | 0.13 | 0.10 | 0.12 | 0.20 | 0.07 | 0.18 |
| Runon (%) | 0.08 | 0.06 | 0.04 | 0.04 | 0.10 | 0.05 | 0.06 |
| Total Reads (M) | 531.96 | 543.67 | 425.26 | 506.04 | 523.59 | 533.46 | 555.07 |
| Q30 decrease | 15.54 | 7.24 | 3.22 | 3.10 | 15.48 | 1.70 | 8.26 |

In the description of this specification, references to terms such as "one embodiment", "some embodiments", "example", "specific example", or "some examples" mean that the specific features, structures, materials, or characteristics described in connection with the embodiment or example are included in at least one embodiment or example of the present disclosure. In this specification, schematic descriptions of the above terms do not necessarily refer to the same embodiment or example. Furthermore, the particular features, structures, materials, or characteristics described may be combined in any suitable manner in any one or more embodiments or examples. Furthermore, combinations and subcombinations of the various embodiments or examples and features of the various embodiments or examples described in this specification can be made by one skilled in the art without departing from the spirit and scope of the present disclosure.

While embodiments of the present disclosure have been shown and described, it is to be understood that the above-described embodiments are illustrative and not restrictive, and that changes, modifications, substitutions, and variations may be made in the above-described embodiments by those of ordinary skill in the art without departing from the scope of the present disclosure.

## Claims

1. A DNA polymerase mutant, comprising:
at least three amino acid mutations at the following four sites or functionally equivalent sites, compared with a *Pyrococcus abyssi* DNA polymerase exo-mutant:
position 409, position 410, position 411, and position 486;
wherein the *Pyrococcus abyssi* DNA polymerase exo-mutant has an amino acid sequence as set forth in SEQ ID NO: 1.

2. The DNA polymerase mutant according to claim 1, wherein the DNA polymerase mutant has at least 90% identity to the *Pyrococcus abyssi* DNA polymerase exo-mutant.

3. The DNA polymerase mutant according to claim 1, having the following mutations:
(1) amino acid L at position 409 is mutated to A, V, Y, H, F, or Q;
(2) amino acid Y at position 410 is mutated to A or G;
(3) amino acid P at position 411 is not mutated or is mutated to V, G, C, or S; and
(4) amino acid A at position 486 is mutated to I, K, L, M, W, or F.

4. The DNA polymerase mutant according to claim 3, having any one of mutation combinations (1) to (2):
(1) amino acid Y at position 410 is mutated to A, amino acid L at position 409 is mutated to A, V, Y, H, F, or Q, amino acid P at position 411 is mutated to V, G, C, or S or is not mutated, and amino acid A at position 486 is mutated to I, K, L, M, W, or F; or
(2) amino acid Y at position 410 is mutated to G, amino acid L at position 409 is mutated to A, V, Y, H, F, or Q, amino acid P at position 411 is mutated to V, G, C, or S or is not mutated, and amino acid A at position 486 is mutated to I, K, L, M, W, or F.

5. The DNA polymerase mutant according to claim 3, having any one of mutation combinations (1) to (7):
(1) amino acid Y at position 410 is mutated to A, amino acid L at position 409 is mutated to A, amino acid P at position 411 is mutated to G, C, or S or is not mutated, and amino acid A at position 486 is mutated to I, K, L, M, W, or F;
(2) amino acid Y at position 410 is mutated to A, amino acid L at position 409 is mutated to V, amino acid P at position 411 is mutated to V, G, or C or is not mutated, and amino acid A at position 486 is mutated to I, L, W, or F;
(3) amino acid Y at position 410 is mutated to A, amino acid L at position 409 is mutated to Y, amino acid P at position 411 is mutated to V or G or is not mutated, and amino acid A at position 486 is mutated to L;
(4) amino acid Y at position 410 is mutated to A, amino acid L at position 409 is mutated to H, amino acid P at position 411 is mutated to V or G or is not mutated, and amino acid A at position 486 is mutated to L;
(5) amino acid Y at position 410 is mutated to A, amino acid L at position 409 is mutated to F, amino acid P at position 411 is mutated to V or G or is not mutated, and amino acid A at position 486 is mutated to L;
(6) amino acid Y at position 410 is mutated to A, amino acid L at position 409 is mutated to Q, amino acid P at position 411 is mutated to G or is not mutated, and amino acid A at position 486 is mutated to L; or
(7) amino acid Y at position 410 is mutated to G, amino acid L at position 409 is mutated to V, amino acid P at position 411 is not mutated, and amino acid A at position 486 is mutated to I, K, M, or F.

6. The DNA polymerase mutant according to claim 4, having any one of mutation combinations (1) to (36):
(1) L at position 409 is mutated to A, Y at position 410 is mutated to A, P at position 411 is not mutated, and A at position 486 is mutated to I;
(2) L at position 409 is mutated to V, Y at position 410 is mutated to A, P at position 411 is not mutated, and A at position 486 is mutated to L;
(3) L at position 409 is mutated to A, Y at position 410 is mutated to A, P at position 411 is not mutated, and A at position 486 is mutated to W;
(4) L at position 409 is mutated to V, Y at position 410 is mutated to G, P at position 411 is not mutated, and A at position 486 is mutated to K;
(5) L at position 409 is mutated to V, Y at position 410 is mutated to G, P at position 411 is not mutated, and A at position 486 is mutated to I;
(6) L at position 409 is mutated to A, Y at position 410 is mutated to A, P at position 411 is not mutated, and A at position 486 is mutated to M;
(7) L at position 409 is mutated to V, Y at position 410 is mutated to G, P at position 411 is not mutated, and A at position 486 is mutated to F;
(8) L at position 409 is mutated to V, Y at position 410 is mutated to A, P at position 411 is mutated to V, and A at position 486 is mutated to L;
(9) L at position 409 is mutated to A, Y at position 410 is mutated to A, P at position 411 is not mutated, and A at position 486 is mutated to F;
(10) L at position 409 is mutated to A, Y at position 410 is mutated to A, P at position 411 is mutated to G, and A at position 486 is mutated to I;
(11) L at position 409 is mutated to A, Y at position 410 is mutated to A, P at position 411 is mutated to C, and A at position 486 is mutated to I;
(12) L at position 409 is mutated to Y, Y at position 410 is mutated to A, P at position 411 is not mutated, and A at position 486 is mutated to L;
(13) L at position 409 is mutated to A, Y at position 410 is mutated to A, P at position 411 is mutated to S, and A at position 486 is mutated to K;
(14) L at position 409 is mutated to Y, Y at position 410 is mutated to A, P at position 411 is mutated to G, and A at position 486 is mutated to L;
(15) L at position 409 is mutated to Y, Y at position 410 is mutated to A, P at position 411 is mutated to V, and A at position 486 is mutated to L;
(16) L at position 409 is mutated to H, Y at position 410 is mutated to A, P at position 411 is not mutated, and A at position 486 is mutated to L;
(17) L at position 409 is mutated to F, Y at position 410 is mutated to A, P at position 411 is not mutated, and A at position 486 is mutated to L;
(18) L at position 409 is mutated to A, Y at position 410 is mutated to A, P at position 411 is not mutated, and A at position 486 is mutated to L;
(19) L at position 409 is mutated to A, Y at position 410 is mutated to A, P at position 411 is mutated to C, and A at position 486 is mutated to M;
(20) L at position 409 is mutated to V, Y at position 410 is mutated to G, P at position 411 is not mutated, and A at position 486 is mutated to M;
(21) L at position 409 is mutated to A, Y at position 410 is mutated to A, P at position 411 is mutated to G, and A at position 486 is mutated to W;
(22) L at position 409 is mutated to A, Y at position 410 is mutated to A, P at position 411 is mutated to G, and A at position 486 is mutated to M;
(23) L at position 409 is mutated to V, Y at position 410 is mutated to A, P at position 411 is not mutated, and A at position 486 is mutated to W;
(24) L at position 409 is mutated to V, Y at position 410 is mutated to A, P at position 411 is mutated to C, and A at position 486 is mutated to L;
(25) L at position 409 is mutated to Q, Y at position 410 is mutated to A, P at position 411 is mutated to G, and A at position 486 is mutated to L;
(26) L at position 409 is mutated to H, Y at position 410 is mutated to A, P at position 411 is mutated to G, and A at position 486 is mutated to L;
(27) L at position 409 is mutated to A, Y at position 410 is mutated to A, P at position 411 is mutated to S, and A at position 486 is mutated to F;
(28) L at position 409 is mutated to V, Y at position 410 is mutated to A, P at position 411 is not mutated, and A at position 486 is mutated to I;
(29) L at position 409 is mutated to H, Y at position 410 is mutated to A, P at position 411 is mutated to V, and A at position 486 is mutated to L;
(30) L at position 409 is mutated to A, Y at position 410 is mutated to A, P at position 411 is mutated to S, and A at position 486 is mutated to I;
(31) L at position 409 is mutated to A, Y at position 410 is mutated to A, P at position 411 is mutated to S, and A at position 486 is mutated to M;
(32) L at position 409 is mutated to F, Y at position 410 is mutated to A, P at position 411 is mutated to G, and A at position 486 is mutated to L;
(33) L at position 409 is mutated to V, Y at position 410 is mutated to A, P at position 411 is mutated to G, and A at position 486 is mutated to L;
(34) L at position 409 is mutated to V, Y at position 410 is mutated to A, P at position 411 is not mutated, and A at position 486 is mutated to F;
(35) L at position 409 is mutated to A, Y at position 410 is mutated to A, P at position 411 is mutated to G, and A at position 486 is mutated to F; or
(36) L at position 409 is mutated to F, Y at position 410 is mutated to A, P at position 411 is mutated to V, and A at position 486 is mutated to L.

7. The DNA polymerase mutant according to claim 1, having the following mutations:
(1) L at position 409 is mutated to V, Y at position 410 is mutated to G, P at position 411 is not mutated, and A at position 486 is mutated to I;
(2) L at position 409 is mutated to A, Y at position 410 is mutated to A, P at position 411 is not mutated, and A at position 486 is mutated to I;
(3) L at position 409 is mutated to A, Y at position 410 is mutated to A, P at position 411 is not mutated, and A at position 486 is mutated to M;
(4) L at position 409 is mutated to A, Y at position 410 is mutated to A, P at position 411 is not mutated, and A at position 486 is mutated to L;
(5) L at position 409 is mutated to V, Y at position 410 is mutated to A, P at position 411 is not mutated, and A at position 486 is mutated to I; or
(6) L at position 409 is mutated to A, Y at position 410 is mutated to A, P at position 411 is mutated to C, and A at position 486 is mutated to F.

8. A nucleic acid molecule, encoding the DNA polymerase mutant according to any one of claims 1 to 7.

9. An expression vector, comprising the nucleic acid molecule according to claim 9.

10. The expression vector according to claim 9, wherein the expression vector is a non-pathogenic viral vector; and
wherein the non-pathogenic viral vector comprises an adenoviral vector or a retroviral vector.

11. A recombinant cell, carrying the nucleic acid molecule according to claim 9 or the expression vector according to claim 10.

12. The recombinant cell according to claim 11, wherein the recombinant cell is selected from *Escherichia coli,* yeast, or mammalian cells.

13. A recombinant strain, expressing the DNA polymerase mutant according to any one of claims 1 to 7.

14. A method for obtaining a DNA polymerase mutant, the method comprising:
culturing the recombinant cell according to any one of claims 11 to 12 or the recombinant strain according to claim 13 under conditions suitable for protein expression, to obtain the DNA polymerase mutant.

15. A complex, comprising the DNA polymerase mutant according to any one of claims 1 to 7 and a small molecule compound or a macromolecule, wherein the mutant is conjugated, via a chemical bond, with the small molecule compound or the macromolecule.

16. The complex according to claim 15, wherein the small molecule compound or the macromolecule comprises a fluorescent label, fluorescein, or an antibody.

17. A method for nucleic acid synthesis, the method comprising:
subjecting a mixture of a nucleic acid template, an amplification primer, dNTPs, and the DNA polymerase mutant according to any one of claims 1 to 7 to amplification under conditions suitable for nucleic acid amplification, to obtain the nucleic acid.

18. A method for nucleic acid sequencing, the method comprising:
subjecting a mixture of a nucleic acid to be sequenced, the DNA polymerase mutant according to any one of claims 1 to 7, and non-natural dNTPs with a fluorescently labeled 3'O-reversible terminator to amplification under conditions suitable for nucleic acid amplification and fluorescence signal detection; and
determining, based on a detected fluorescence signal, the nucleotide sequence of the nucleic acid to be sequenced.

19. A nucleic acid sequencing kit, comprising the DNA polymerase mutant according to any one of claims 1 to 7 or the complex according to claim 15 or 16.

20. Use of the nucleic acid sequencing kit according to claim 19 in sequencing.

21. Use of the DNA polymerase mutant according to any one of claims 1 to 7, the nucleic acid molecule according to claim 8, the expression vector according to any one of claims 9 to 10, the recombinant cell according to any one of claims 11 to 12, the recombinant strain according to claim 13, or the complex according to any one of claims 15 to 16 in the preparation of a product for catalyzing DNA amplification or nucleic acid sequencing.
